# EUROPEAN PATENT APPLICATION

(11) **EP 1 903 039 A1**
(43) Date of publication of application: **26.03.2008**
(21) Application number: 06757247.9
(22) Date of filing: 12.06.2006
(51) Int. Cl.: C07D 401/12

(54) **INJECTION**

(30) Priority: 13.06.2005 JP 2005173039
(71) Applicant: Takeda Pharmaceutical Company Limited, Osaka-shi, Osaka 541-0045 (JP)
(72) Inventor: NAKAI, S., c/o Takeda Pharmaceutical Company Ltd., Osaka-shi, Osaka 532-8686 (JP); INOUE, T., c/o Takeda Pharmaceutical Company Ltd., Osaka-shi, Osaka 532-8686 (JP)
(74) Representative: Rickard, Timothy Mark Adrian
(86) International application number: PCT/JP2006/311762
(87) International publication number: WO 2006/134877

(57) **Abstract**

In the case of storing and supplying an injection containing a 2-[(2-pyridyl)methylsulfinyl]benzimidazole type compound in a plastic container in addition to a glass container, an injection having been improved in stability and solubility and showing excellent qualities without forming any insoluble foreign matters or insoluble microparticles can be obtained by using cyclodextrin or its derivative together.

## Description

### Technical Field

The present invention relates to injections containing a benzimidazole compound such as lansoprazole having an anti-ulcer action, and methods of using them.

### Background Art

As injections comprising a 2-[(2-pyridinyl)methylsulfinyl]benzimidazole compound having an anti-ulcer action, for example, the following injections have been reported.
1) Patent Document 1 (JP-A-2-138213) discloses an injection which comprises a benzimidazole compound having an anti-ulcer action and at least one of ethanol, propylene glycol and polyethylene glycol. The document also discloses an injectable solution which contains a freeze-dried product of the benzimidazole compound dissolved in a mixture of an acidic substance and polyethylene glycol, and further contains a saccharide such as mannitol, and N-methylglucamine.
2) Patent Document 2 (JP-A-2002-128675) discloses an injection using a strong alkali in 1:1 of a molar ratio relative to 2-[(2-pyridinyl)methylsulfinyl]benzimidazole compound having an anti-ulcer action so that the amount of an alkali to be used is as small as possible, that a pain or a local irritation can be suppressed, that the kneading operation and the complicated dissolving operation are not required, that the injection can be dissolved by a simple operation, and further that it is not necessary to attach any special dissolving solution.

An injection containing 2-[(2-pyridinyl)methylsulfinyl]benzimidazole compound is used in therapy by injecting intravenously the solution of the injection which is mixed with physiological saline, 5% glucose solution, or the like. In that case, although previously a glass container has been predominantly used as a container for an infusion solution, a plastic container is now in a main use. The plastic container includes a container made of polyethylene or polypropylene as a hard type, a container made of the same material as a relatively soft type, a container made of polyvinyl chloride or an ethylene-vinyl acetate copolymer as a softer type, and the like. It is known that different additives such as a mold releasing agent and a catalyst are used when different plastic containers are manufactured by different manufacturers. In the European Pharmacopoeia, the material for a plastic container for an injectable infusion is provided that the concentration of the ions of the metals such as aluminum, zinc or titanium which is eluted when 100 g of the material for the container is boiled and refluxed with hydrochloric acid for 1 hour is not more than 1 ppm. However, there is no similar provision for commercially available infusions in U.S.A., etc., and it is recognized that the amount of the metallic ion to be eluted is high for some containers for commercially available infusions in many countries in the world.

The European Pharmacopoeia provides that the content of zinc stearate or calcium stearate, or a mixture thereof in the material (film) for a plastic container is not more than 0.5% (in polyolefin, polyethylene) and not more than 1% (in polyvinyl chloride), and the content of fatty acid or a salt thereof is not more than 0.5% (in polyolefin, polyethylene). Generally, problems that components in the material for the plastic container for injection are eluted from the container to form foreign insoluble matters, insoluble particulate matters and the like may occur.
Patent Document 1: Japanese published unexamined application No. 2-138213
Patent Document 2: Japanese published unexamined application No. 2002-128675

### Summary of Invention

The object of the present invention is to provide high-quality injections having higher stability and solubility in which foreign insoluble matter, insoluble particulate matters or the like are not formed even when the injection containing a 2-[(2-pyridinyl)methylsulfinyl]benzimidazole compound is kept and supplied in a plastic container as well as in a conventionally-used glass containers.

The present inventors have found, as a result of intensive studies for solving the above problems, that when 2-[(2-pyridinyl)methylsulfinyl]benzimidazole compound having an anti-ulcer action is used in combination with cyclodextrin or derivatives thereof in an amount corresponding to about 0.16- to about 170-fold by weight, preferably about 0.16- to about 100-fold by weight relative to a pharmaceutically active ingredient such as lansoprazole or its optically active compound or a salt thereof, the formation of foreign insoluble matters or insoluble particulate matters from fatty acid salts such as stearate eluted from a plastic container can be suppressed, and as a result, the injection containing the benzimidazole compound can be filled not only in a glass container but also in a plastic bag such as an infusion bag, or a plastic vial, kept therein and supplied therefrom. The present inventors have further studied based on the findings and accomplished the present invention.

As used herein, the "foreign insoluble matters" refer to foreign matters as detected in accordance with the Japanese Pharmacopoeia, General Tests, Foreign Insoluble Matter Test for Injection, and the "insoluble particulate matters" refer to particles as detected in accordance with the Japanese Pharmacopoeia, General Tests, Insoluble Particulate Matter Test for Injection, Method 1, Light Obscuration Particle Count Test.

That is, the present invention relates to:
(1) an injection comprising a compound represented by formula (I): 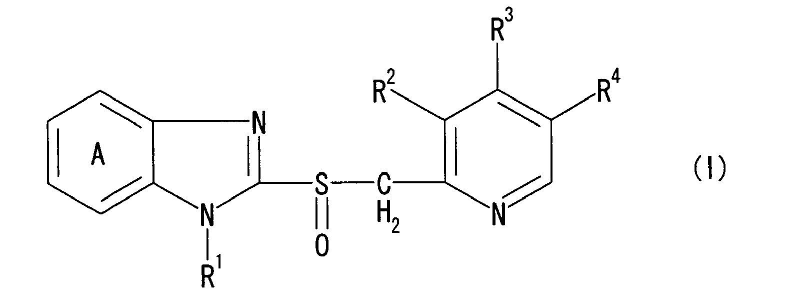 wherein the ring A is a benzene or pyridine ring which may have a substituent, R¹ represents a hydrogen atom, an aralkyl group which may have a substituent, an acyl group or an acyloxy group, and R², R³ and R⁴ are the same or different and each represent a hydrogen atom, an alkyl group which may have a substituent, an alkoxy group which may have a substituent, or an amino group which may have a substituent, or its optically active compound or a salt thereof, in combination with a sulfobutylated derivative of β-cyclodextrin or a salt thereof, wherein pH of the injection is 8 to 12 when used;
(2) the injection according to the above-mentioned (1), wherein the compound of the formula (I) is 2-[[[3-methyl-4-(2,2,2-trifluoroethoxy)-2-pyridinyl]methyl]sulfinyl]-1H-benzimidazole (lansoprazole), its optically active compound or a salt thereof;
(3) the injection according to the above-mentioned (1), further comprising a chelating agent;
(4) the injection according to the above-mentioned (1), wherein pH of the injection is 9 to 12 when used;
(5) the injection according to the above-mentioned (1), which comprises about 0.5 to about 3 equivalents of a strong alkali per mol of the compound of the formula (I);
(6) the injection according to the above-mentioned (1), further comprising N-methylglucamine;
(7) the injection according to the above-mentioned (6), wherein the amount of N-methylglucamine is about 0.1 mg to about 1 mg per mg of the compound of the formula (I);
(8) an injection substantially free from insolubles which is filled in a container wherein the injection comprises a solution of a compound represented by formula (I): 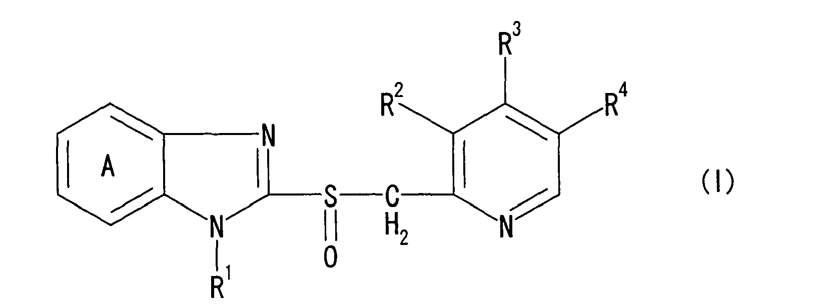 wherein the ring A is a benzene or pyridine ring which may have a substituent, R¹ represents a hydrogen atom, an aralkyl group which may have a substituent, an acyl group or an acyloxy group, and R², R³ and R⁴ are the same or different and each represent a hydrogen atom, an alkyl group which may have a substituent, an alkoxy group which may have a substituent, or an amino group which may have a substituent, or its optically active compound or a salt thereof, and a sulfobutylated derivative of β-cyclodextrin or a salt thereof;
(9) the injection according to the above-mentioned (8), wherein the compound of the formula (I) and the sulfobutylated derivative of β-cyclodextrin or a salt thereof are separately kept, and are mixed with each other when used;
(10) the injection according to the above-mentioned (8), wherein the container is made of polyethylene, polypropylene, a polyethylene-polypropylene copolymer, polyvinyl chloride, an ethylene-vinyl acetate copolymer, an ethylene-propylene copolymer, silicone, polybutadiene, a thermoplastic elastomer, Teflon (Registered Trademark), polyurethane, cyclic polyolefin or polyolefin;
(11) the injection according to the above-mentioned (1), which comprises the sulfobutylated derivative of β-cyclodextrin or a salt thereof in an amount corresponding to about 0.16- to about 170-fold by weight relative to the compound of the formula (I);
(12) the injection according to the above-mentioned (3), wherein the chelating agent consists of edetic acid, its salt or a derivative thereof, phosphoric acid or a salt thereof, or citric acid or a salt thereof;
(13) the injection according to the above-mentioned (3), wherein the chelating agent is a sodium salt of edetic acid;
(14) the injection according to the above-mentioned (3), wherein as the chelating agent, edetic acid or its salt is contained in an amount corresponding to about 0.03% to about 67% by weight relative to the compound of the formula (I) ;
(15) the injection according to the above-mentioned (1), which is a lyophilized preparation;
(16) the injection according to the above-mentioned (1), which further comprises a saccharide;
(17) the injection according to the above-mentioned (16), wherein the saccharide is a sugar alcohol;
(18) the injection according to the above-mentioned (16), wherein the saccharide is mannitol;
(19) the injection according to the above-mentioned (16), wherein the amount of the saccharide is about 0.1 mg to about 20 mg per mg of the compound of the formula (I);
(20) the injection according to the above-mentioned (2), which comprises about 5 to 5000 mg of the sulfobutylated derivative of β-cyclodextrin or a salt thereof, about 1.5 to about 10 mg of sodium hydroxide, about 8 to about 24 mg of N-methylglucamine, and about 0.009 to about 20.1 mg of disodium edetate per 30 mg of lansoprazole, its optically active compound or a salt thereof;
(21) the injection according to the above-mentioned (2), which comprises about 5 to 5000 mg of the sulfobutylated derivative of β-cyclodextrin or a salt thereof, about 1.5 to about 10 mg of sodium hydroxide, about 8 to about 24 mg of N-methylglucamine, about 10 to about 100 mg of mannitol and about 0.009 to about 20.1 mg of disodium edetate per 30 mg of lansoprazole, its optically active compound or a salt thereof;
(22) an injection which is prepared by adding an aqueous or a non-aqueous solvent containing a sulfobutylated derivative of β-cyclodextrin or a salt thereof and edetic acid or its salt to a lyophilized injection containing 30 mg of lansoprazole, its optically active compound or a salt thereof, about 1.5 to about 10 mg of sodium hydroxide, about 8 to about 24 mg of N-methylglucamine and 60 mg of mannitol;
(23) the injection according to the above-mentioned (1), which is an agent for prevention or treatment of peptic ulcer, gastroesophageal reflux disease; gastritis; Zollinger-Ellison syndrome; NUD (Non Ulcer Dyspepsia); gastric cancer; gastric MALT lymphoma; upper gastrointestinal hemorrhage due to gastric ulcer, duodenal ulcer, acute stress ulcer and acute gastric mucosal lesion, ulcer caused by a nonsteroidal anti-inflammatory agent; gastric hyperacidity and ulcer due to postoperative stress; upper gastrointestinal hemorrhage due to invasive stress; atrophic gastritis after endoscopic mucosal resection (EMR) for early gastric cancer; hyperplastic gastric polyp; idiopathic thrombocytopenic purpura; a disease caused by *Helicobacter pylori*; asthma caused by gastric acid reflux, sleep disorder due to gastric acid reflux; abdominal pain due to GERD; laryngitis; chronic obstructive pulmonary disease (COPD); obstructive apnea; or Barrett's esophagus;
(24) a method for preventing or treating peptic ulcer, gastroesophageal reflux disease; gastritis; Zollinger-Ellison syndrome; NUD (Non Ulcer Dyspepsia); gastric cancer; gastric MALT lymphoma; upper gastrointestinal hemorrhage due to gastric ulcer, duodenal ulcer, acute stress ulcer and acute gastric mucosal lesion, ulcer caused by a nonsteroidal anti-inflammatory agent; gastric hyperacidity and ulcer due to postoperative stress; upper gastrointestinal hemorrhage due to invasive stress; atrophic gastritis after endoscopic mucosal resection (EMR) for early gastric cancer; hyperplastic gastric polyp; idiopathic thrombocytopenic purpura; a disease caused by *Helicobacter pylori;* asthma caused by gastric acid reflux, sleep disorder due to gastric acid reflux; abdominal pain due to GERD; laryngitis; chronic obstructive pulmonary disease (COPD); obstructive apnea; or Barrett's esophagus, which comprises administering the injection according to the above-mentioned (1) to humans;
(25) use of the injection according to the above-mentioned
   (1) for an agent for prevention or treatment of peptic ulcer, gastroesophageal reflux disease; gastritis; Zollinger-Ellison syndrome; NUD (Non Ulcer Dyspepsia); gastric cancer; gastric MALT lymphoma; upper gastrointestinal hemorrhage due to gastric ulcer, duodenal ulcer, acute stress ulcer and acute gastric mucosal lesion, ulcer caused by a nonsteroidal anti-inflammatory agent; gastric hyperacidity and ulcer due to postoperative stress; upper gastrointestinal hemorrhage due to invasive stress; atrophic gastritis after endoscopic mucosal resection (EMR) for early gastric cancer; hyperplastic gastric polyp; idiopathic thrombocytopenic purpura; a disease caused by *Helicobacter pylori;* asthma caused by gastric acid reflux, sleep disorder due to gastric acid reflux; abdominal pain due to GERD; laryngitis; chronic obstructive pulmonary disease (COPD); obstructive apnea; or Barrett's esophagus; and
(26) a method of suppressing the generation of foreign insoluble matters and insoluble particulate matters in an injection filled in a container, which comprises incorporating cyclodextrin or a derivative thereof into the injection, wherein pH of the injection is 7 to 12 when used, and the container is made of polyethylene, polypropylene, a polyethylene-polypropylene copolymer, polyvinyl chloride, an ethylene-vinyl acetate copolymer, an ethylene-propylene copolymer, silicone, polybutadiene, a thermoplastic elastomer, Teflon (Registered Trademark), polyurethane, cyclic polyolefin or polyolefin.

The injection of the present invention can be provided as a high-quality injection in which foreign insoluble matters, insoluble particulate matters or the like are formed even when the injection containing a 2-[(2-pyridinyl)methylsulfinyl]benzimidazole compound such as lansoprazole, its optically active compound or a salt thereof is stored, kept or supplied in not only a glass container but also a plastic container, or even when the injectable solution is kept for a long time in such container after preparation of the injectable solution. The present invention can be also applied to an injection comprising an active ingredient other than lansoprazole as an active pharmaceutical ingredient, wherein pH of the injection is about 7 to about 12 when used, and a plastic container is used as a container for the injection, and the generation of foreign insoluble matters and insoluble particulate matters can be suppressed by incorporating cyclodextrin or a derivative thereof (particularly a sulfobutytated derivative of β-cyclodextrin or a salt thereof) into the injection.

Further, by incorporating cyclodextrin or a derivative thereof (particularly a sulfobutytated derivative of β-cyclodextrin or a salt thereof) into the injection, the decomposition rate of the 2-[(2-pyridinyl)methylsulfinyl]benzimidazole compound in the solution is decreased thus contributing to improvement in stability as well.

### Best Mode for Carrying Out the Invention

The active ingredient used in the present invention includes a compound represented by the following formula (I), or its optically active compound or a salt thereof: 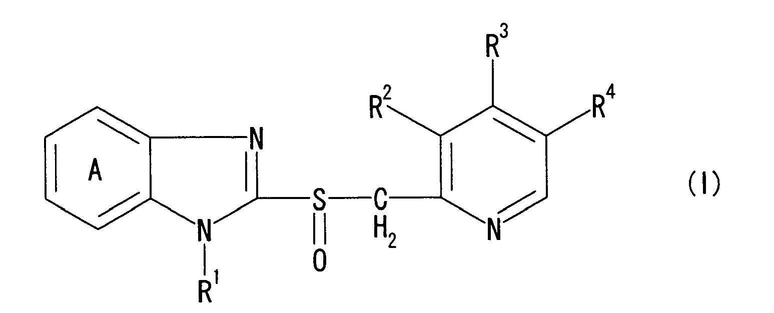 wherein the ring A is a benzene or pyridine ring which may have a substituent, R¹ represents a hydrogen atom, an aralkyl group which may have a substituent, an acyl group or an acyloxy group, R², R³ and R⁴ are the same or different and each represent a hydrogen atom, an alkyl group which may have a substituent, an alkoxy group which may have a substituent, or an amino group which may have a substituent.

Preferable compounds of the formula (I) are those wherein the ring A represents a benzene or pyridine ring which may have a substituent selected from a halogen atom, an optionally halogenated C₁₋₄ alkyl group, an optionally halogenated C₁₋₄ alkoxy group and a 5- or 6-memberred heterocyclic group, R¹ represents a hydrogen atom, R² represents a C₁₋₆ alkyl group, a C₁₋₆ alkoxy group, a C₁₋₆ alkoxy-C₁₋₆ alkoxy group or a di-C₁₋₆ alkylamino group, R³ represents a hydrogen atom, a C₁₋₆ alkoxy-C₁₋₆ alkoxy group or an optionally halogenated C₁₋₆ alkoxy group, and R⁴ represents a hydrogen atom or a C₁₋₆ alkyl group.

The "substituent" in the "benzene or pyridine ring which may have a substituent" represented by the ring A in the compound represented by the above formula (I) includes, for example, a halogen atom, a cyano group, a nitro group, an alkyl group which may have a substituent, a hydroxy group, an alkoxy group which may have a substituent, an aryl group, an aryloxy group, a carboxy group, an acyl group, an acyloxy group, and a 5- to 10-memberred heterocyclic group, and the benzene or pyridine ring may be substituted with one to three or so of these substituents. When the number of substituents is 2 or more, the respective substituents may be the same or different. Among these substituents, a halogen atom, an alkyl group which may have a substituent and an alkoxy group which may have a substituent are preferable.

The halogen atom includes a fluorine atom, a chlorine atom, a bromine atom, etc. Among these, fluorine is preferable.

In the "alkyl group which may have a substituent", the "alkyl group" includes, for example, a C₁₋₇ alkyl group (for example, a methyl group, an ethyl group, a propyl group, an isopropyl group, a butyl group, an isobutyl group, a sec-butyl group, a tert-butyl group, a pentyl group, a hexyl group, and a heptyl group). In the "alkyl group which may have a substituent", the "substituent" can be exemplified by a halogen atom, a hydroxy group, a C₁₋₆ alkoxy group (for example, methoxy, ethoxy, propoxy, and butoxy), a C₁₋₆ alkoxy-carbonyl group (for example, a methoxycarbonyl group, an ethoxycarbonyl group, and a propoxycarbonyl group), and a carbamoyl group, and the number of these substituents may be 1 to 3 or so. When the number of substituents is 2 or more, the respective substituents may be the same or different.

In the "alkoxy group which may have a substituent", the "alkoxy" group includes, for example, a C₁₋₆ alkoxy group (for example, methoxy, ethoxy, propoxy, isopropoxy, butoxy, isobutoxy, and pentoxy). In the "alkoxy group which may have a substituent", the "substituent" can be exemplified by the same substituents as exemplified for the "substituent" in the "alkyl group which may have a substituent", and the number of substituents is the same as defined therein.

The "aryl group" includes, for example, a C₆₋₁₄ aryl group (for example, a phenyl group, a 1-naphthyl group, a 2-naphthyl group, a biphenyl group, and a 2-anthryl group).

The "aryloxy group" includes, for example, a C₆₋₁₄ aryloxy group (for example, a phenyloxy group, 1-naphthyloxy group, and a 2-naphthyloxy group).

The "acyl group" includes, for example, a formyl group, an alkylcarbonyl group, an alkoxycarbonyl group, a carbamoyl group, an alkylcarbamoyl group, an alkylsulfinyl group, and an alkylsulfonyl group.

The "alkylcarbonyl group" includes a C₁₋₆ alkylcarbonyl group (for example, an acetyl group, and a propionyl group).

The "alkoxycarbonyl group" includes, for example, a C₁₋₆ alkoxy-carbonyl group (for example, a methoxycarbonyl group, an ethoxycarbonyl group, a propoxycarbonyl group, and a butoxycarbonyl group).

The "alkylcarbamoyl group" includes an N-C₁₋₆ alkyl-carbamoyl group (for example, a methylcarbamoyl group, and an ethylcarbamoyl group), and an N,N-di-C₁₋₆ alkyl-carbamoyl group (for example, an N,N-dimethylcarbamoyl group, and an N,N-diethylcarbamoyl group).

The "alkylsulfinyl group" includes, for example, a C₁₋₇ alkylsulfinyl group (for example, a methylsulfinyl group, an ethylsulfinyl group, a propylsulfinyl group, and an isopropylsulfinyl group).

The "alkylsulfonyl group" includes, for example, a C₁₋₇ alkylsulfonyl group (for example, a methylsulfonyl group, an ethylsulfonyl group, a propylsulfonyl group, and an isopropylsulfonyl group).

The "acyloxy" group includes, for example, an alkylcarbonyloxy group, an alkoxycarbonyloxy group, a carbamoyloxy group, an alkylcarbamoyloxy group, an alkylsulfinyloxy group, and an alkylsulfonyloxy group.

The "alkylcarbonyloxy group" includes a C₁₋₆ alkylcarbonyloxy group (for example, an acetyloxy group, and a propionyloxy group).

The "alkoxycarbonyloxy group" includes, for example, a C₁₋₆ alkoxy-carbonyloxy group (for example, a methoxycarbonyloxy group, an ethoxycarbonyloxy group, a propoxycarbonyloxy group, and a butoxycarbonyloxy group).

The "alkylcarbamoyloxy group" includes a C₁₋₆ alkylcarbamoyloxy group (for example, a methylcarbamoyloxy group, and an ethylcarbamoyloxy group).

The "alkylsulfinyloxy group" includes, for example, a C₁₋₇ alkylsulfinyloxy group (for example, a methylsulfinyloxy group, an ethylsulfinyloxy group, a propylsulfinyloxy group, and an isopropylsulfinyloxy group).

The "alkylsulfonyloxy group" includes, for example, a C₁₋₇ alkylsulfonyloxy group (for example, a methylsulfonyloxy group, an ethylsulfonyloxy group, a propylsulfonyloxy group, and an isopropylsulfonyloxy group).

The "5- to 10-memberred heterocyclic group" includes, for example, a 5- to 10-memberred (preferably 5- or 6-memberred) heterocyclic group containing 1 or more (preferably 1 to 3) heteroatoms selected from a nitrogen atom, a sulfur atom and an oxygen atom in addition to carbon atoms, and specific examples include a 2- or 3-thienyl group, a 2-, 3- or 4-pyridyl group, a 2- or 3-furyl group, a 1-, 2- or 3-pyrrolyl group, a 2-, 3-, 4-, 5- or 8-quinolyl group, a 1-, 3-, 4- or 5-isoquinolyl group, and a 1-, 2- or 3-indolyl group. Among these groups, a 5- or 6-memberred heterocyclic group such as a 1-, 2- or 3-pyrrolyl group is preferable.

The ring A is preferably a benzene or pyridine ring which may have 1 or 2 substituents selected from a halogen atom, an optionally halogenated C₁₋₄ alkyl group, an optionally halogenated C₁₋₄ alkoxy group and a 5- or 6-memberred heterocyclic group.

In the "aralkyl group which may have a substituent" represented by R¹, the "aralkyl group" includes, for example, a C₇₋₁₆ aralkyl group (for example, a C₆₋₁₀ aryl-C₁₋₆ alkyl group such as benzyl and phenethyl). In the "aralkyl group which may have a substituent", the "substituent" can be exemplified by the same substituents as exemplified for the "substituent" in the "alkyl group which may have a substituent", and the number of substituents is 1 to 4 or so. When the number of substituents is 2 or more, the respective substituents may be the same or different.

The "acyl group" represented by R¹ includes, for example, the "acyl group" described as a substituent on the ring A.

The "acyloxy group" represented by R¹ includes, for example, the "acyloxy group" described as a substituent on the ring A.

R¹ is preferably a hydrogen atom.

The "alkyl group which may have a substituent" represented by R², R³ or R⁴ includes the "alkyl group which may have a substituent" described as a substituent on the ring A.

The "alkoxy group which may have a substituent" represented by R², R³ or R⁴ includes the "alkoxy group which may have a substituent" described as a substituent on the ring A.

The "amino group which may have a substituent" represented by R², R³ or R⁴ includes, for example, an amino group, a mono-C₁₋₆ alkylamino group (for example, methylamino, and ethylamino), a mono-C₆₋₁₄ arylamino group (for example, phenylamino, 1-naphthylamino, and 2-naphthylamino), a di-C₁₋₆ alkylamino group (for example, dimethylamino, and diethylamino), and a di-C₆₋₁₄ arylamino group (for example, diphenylamino).

R² is preferably a C₁₋₆ alkyl group, a C₁₋₆ alkoxy group, a C₁₋₆ alkoxy-C₁₋₆ alkoxy group or a di-C₁₋₆ alkylamino group. R² is more preferably a C₁₋₃ alkyl group or a C₁₋₃ alkoxy group.

R³ is preferably a hydrogen atom, a C₁₋₆ alkoxy-C₁₋₆-alkoxy group or an optionally halogenated C₁₋₆ alkoxy group. R³ is more preferably a C₁₋₃ alkoxy group which is halogenated or optionally substituted with a C₁₋₃ alkoxy group.

R⁴ is preferably a hydrogen atom or a C₁₋₆ alkyl group. R⁴ is preferably a hydrogen atom or a C₁₋₃ alkyl group (particularly a hydrogen atom).

Specific examples of the compound represented by the formula (I) include:
2-[[[3-methyl-4-(2,2,2-trifluoroethoxy)-2-pyridinyl]methyl]sulfinyl]-1H-benzimidazole,
2-[[(3,5-dimethyl-4-methoxy-2-pyridinyl)methyl]sulfinyl]-5-methoxy-1H-benzimidazole,
2-[[[4-(3-methoxypropoxy)-3-methyl-2-pyridinyl]methyl]sulfinyl]-1H-benzimidazole sodium salt,
5-difluoromethoxy-2-[[(3,4-dimethoxy-2-pyridinyl)methyl]sulfinyl]-1H-benzimidazole, and
2-[(RS)-[(4-methoxy-3-methylpyridin-2-yl)methyl]sulfinyl]-5-(1H-pyrrol-1-yl)-1H-benzimidazole.

As more specific examples of the compound represented by the formula (I) in the present invention, it is preferable to use benzimidazole compounds and imidazopyridine compounds such as lansoprazole, omeprazole, esomeprazole, pantoprazole, rabeprazole, tenatoprazole and ilaprazole.

Among these compounds, lansoprazole, that is, 2-[[[3-methyl-4-(2,2,2-trifluoroethoxy)-2-pyridinyl]methyl]sulfinyl]-1H-benzimidazole is particularly preferable.

The compound (I) may be a racemic compound or an optically active compound such as R-form and S-form. For example, the compound (I) may be an optically active compound of lansoprazole, that is, R-form or S-form of lansoprazole. Particularly, an optically active compound such as (R)-2-[[[3-methyl-4-(2,2,2-trifluoroethoxy)-2-pyridinyl]methyl]sulfinyl]-1H-benzimidazole is preferable.

The active ingredient may be a pharmaceutically acceptable salt of the compound represented by the formula (I) or of its optically active compound. The salt is preferably a pharmaceutically acceptable salt and includes, for example, a salt with an inorganic base, a salt with an organic base, and a salt with a basic amino acid.

Preferred examples of the salt with an inorganic base include, for example, an alkali metal salt such as a sodium salt and a potassium salt; an alkaline earth metal salt such as a calcium salt and a magnesium salt; and an ammonium salt.

Preferred examples of the salt with an organic base include, for example, a salt with an alkylamine (e.g., trimethylamine, triethylamine), a heterocyclic amine (e.g., pyridine, picoline), an alkanolamine (e.g., ethanolamine, diethanolamine, triethanolamine), dicyclohexylamine, or N,N'-dibenzylethylenediamine.

Preferred examples of the salt with a basic amino acid include, for example, a salt with arginine, lysine, or ornithine.

Among these salts, the alkali metal salt or the alkaline earth metal salt is preferable. In particular, the sodium salt is preferable.

The compound represented by the formula (I) can be prepared by methods known per se, for example, the methods described in JP-A 61-50978, U.S. Pat. No. 4,628,098, JP-A 10-195068, WO 98/21201 or methods based on these methods. The optically active compound represented by the formula (I) can be obtained by an optical resolution method (e.g., a fractional recrystallization method, a chiral column method, a diastereomer method, a method with a microorganism or an enzyme), an asymmetric oxidation method. R-lansoprazole, for example, can be prepared by methods described in WO 00/78745, WO 01/83473, WO 01/87874 and WO 02/44167.

The injection of the present invention is characterized in that the active ingredient described above is used in combination with cyclodextrin or a derivative thereof. Cyclodextrin or a derivative thereof may previously be incorporated into the active ingredient, if desired, together with other components, and supplied, or alternatively, cyclodextrin or a derivative thereof may be kept separately from a preparation containing the active ingredient, and may be mixed with the preparation to prepare an injection when used.

Cyclodextrin or a derivative thereof includes, for example, α-cyclodextrin, β-cyclodextrin, γ-cyclodextrin and derivatives thereof (which may be salts such as alkali metal salts (for example, sodium salts), and these may be used alone or as a mixture of two or more thereof. In particular, derivatives of β-cyclodextrin, such as sulfobutyrated derivatives (for example, sulfobutylether-β-cyclodextrin (trade name: CAPTISOL, manufactured by CyDex Inc.)), hydroxypropylated derivatives (for example, hydroxypropyl-β-cyclodextrin) and maltosylated derivatives (for example, maltosyl-β-cyclodextrin) are preferable. A high-quality injection can be provided by incorporating cyclodextrin or a derivative thereof in an amount corresponding to about 0.16- to about 170-fold by weight, preferably about 0.16- to about 100-fold by weight relative to the pharmaceutically active ingredient such as the compound represented by the formula (I) such as lansoprazole, its optically active compound or a salt thereof.

The present invention relates to an injection comprising the compound represented by the formula (I) such as lansoprazole, its optically active compound or a salt thereof in combination with cyclodextrin or a derivative thereof, wherein cyclodextrin or a derivative thereof suppresses the formation of foreign insoluble matters or insoluble particulate matters from fatty acid salts such as stearate.

The injection of the present invention may further comprise a chelating agent for the purpose of inhibiting formation of foreign insoluble matters and insoluble particulate matters which are formed from a metal ion eluted from a container and the compound represented by the formula (I) such as lansoprazole, its optically active compound or a salt thereof.

The chelating agent forms a chelate compound (a complex compound with a metal ion) with a metal ion eluted from a container for an infusion solution or the like, thereby inhibiting formation of foreign insoluble matters and insoluble particulate matters which are formed from the metal ion and the compound represented by the formula (I) such as lansoprazole, its optically active compound or a salt thereof.

Therefore, the present invention includes an injection comprising the compound represented by the formula (I) such as lansoprazole, its optically active compound or a salt thereof, cyclodextrin or a derivative thereof, and a chelate compound.

The chelating agent includes, for example, edetic acid, its salt or a derivative thereof, phosphoric acid or its salt, and citric acid or its salt, and can be used alone or as a mixture of two or more thereof. Particularly, edetic acid or its salt is preferable. For example, an injection which contains edetic acid or its salt in an amount corresponding to about 0.03% to about 67% by weight, preferably about 0.3% to about 33% by weight, more preferably about 0.6% to about 6.7% by weight relative to the compound represented by the formula (I) such as lansoprazole, its optically active compound or a salt thereof, can be provided as a high-quality injection. As a preferable salt of edetic acid, a salt with sodium or calcium, a mixed salt thereof or the like can be preferably used. That is, the preferable salt includes a sodium salt, a calcium salt, or a sodium and calcium salt (calcium disodium edetate or the like) of edetic acid. In particular, sodium salts of edetic acid such as disodium edetate, tetrasodium edetate and calcium disodium edetate are preferable, and disodium edetate is particularly preferable. Usually, edetic acid or its salt may be used in an amount corresponding to about 0.03% to about 67% by weight relative to the compound represented by the formula (I) such as lansoprazole, its optically active compound or a salt thereof.

As the container for the injection, various containers regardless of materials for the containers such as glass containers and plastic containers can be used. As the plastic container, containers made of polyethylene, polypropylene, a polyethylene-polypropylene copolymer, polyvinyl chloride, an ethylene-vinyl acetate copolymer, an ethylene-propylene copolymer, silicone, polybutadiene, a thermoplastic elastomer, Teflon (Registered Trademark), polyurethane, cyclic polyolefin or polyolefin can be used.

In the injection of the present invention, the compound represented by the formula (I) such as lansoprazole, its optically active compound or a salt thereof and cyclodextrin or a derivative thereof may be contained in the same container, or may be filled in separate containers and mixed with each other when used. Alternatively, the compound represented by the formula (I), its optically active compound or a salt thereof is sealed into one chamber of an infusion bag whose inside is separated into two chambers by a separator, and an infusion solution is sealed into the other chamber, and cyclodextrin or a derivative thereof may be sealed into either of the two chambers.

When a chelating agent is used, the chelating agent may be contained in the same container as the container in which the compound represented by the formula (I) such as lansoprazole, its optically active compound or a salt thereof and cyclodextrin or a derivative thereof are contained, or may be filled in a container different from the container in which the compound represented by the formula (I) such as lansoprazole, its optically active compound or a salt thereof and cyclodextrin or a derivative thereof are contained and mixed with each other when used. Alternatively, if the compound represented by the formula (I), its optically active compound or a salt thereof is sealed into one chamber of an infusion bag whose inside is separated into two chambers by a separator, and an infusion solution is sealed into the other chamber, the chelating agent may be sealed into either of the two chambers.

The compound represented by the formula (I) such as lansoprazole, its optically active compound or a salt thereof may be in a form of a liquid injection or a solid injection such as a lyophilized injection or a powdery injection. The solid injection can be dissolved in or diluted with a solvent which is substantially free from a non-aqueous solvent.

Usually, the injection of the present invention can be dissolved in or diluted with a solvent which is substantially free from any non-aqueous solvent (or a water-soluble organic solvent) and whose medium is substantially water by incorporating a strong alkali in addition to the compound represented by the formula (I) such as lansoprazole, its optically active compound or a salt thereof and cyclodextrin or a derivative thereof (or preferably cyclodextrin or a derivative thereof and a chelating agent) in the injection. The strong alkali is incorporated in such an amount that pH of the injection is about 7 to about 12, preferably about 8 to about 12, more preferably about 9 to about 12 when used, and is usually about 0.5 to about 3 equivalents per mol of the compound represented by the formula (I) such as lansoprazole or its optically active compound, though the amount of the strong alkali varies depending on the kind and amount of cyclodextrin or a derivative thereof to be used.

When 30 mg of the compound represented by the formula (I), its optically active compound or a salt thereof, and cyclodextrin or a derivative thereof, are dissolved in 5 ml of physiologic saline or distilled water for injection, the resulting solution preferably has a pH value of about 9 to 12 or so, particularly a pH value of about 9.5 to about 12.0 or so.

The injection of the present invention may further contain N-methylglucamine so as to suppress the lowering of pH and to stabilize the solubility when an injection is prepared. The amount of N-methylglucamine to be incorporated may be about 0.1 mg to about 1 mg or so per mg of the compound represented by the formula (I) such as lansoprazole, its optically active compound or a salt thereof. Further, when the injection is prepared as the solid preparation, the injection may contain a saccharide (e.g., a sugar alcohol such as mannitol) so as to stabilize the shape of the solid preparation to be prepared. The amount of the saccharide to be incorporated may be about 0.1 mg to about 20 mg per mg of the compound represented by the formula (I), its optically active compound or a salt thereof. The injection containing such ingredients is an injection comprising the compound represented by the formula (I), its optically active compound or a salt thereof and capable of being dissolved in or diluted with a solvent substantially free from a non-aqueous solvent wherein the injection may contain about 0.1 mg to about 0.8 mg of N-methylglucamine and about 1 mg to about 10 mg of a sugar alcohol per mg of the compound represented by the formula (I), its optically active compound or a salt thereof.

Moreover, the injection is preferably an injection comprising about 5 mg to about 5 g of sulfobutylether-β-cyclodextrin, hydroxypropyl-β-cyclodextrin, maltosyl-β-cyclodextrin or a mixture thereof; about 0.009 mg to about 20.1 mg of disodium edetate, tetrasodium edetate, calcium disodium edetate or a mixture thereof; about 8 mg to about 24 mg of N-methylglucamine; and about 1.5 mg to about 10 mg of sodium hydroxide per 30 mg of the compound represented by the formula (I) such as lansoprazole, its optically active compound or a salt thereof. An injection in which about 10 mg to about 100 mg (preferably about 50 to about 70 mg) of mannitol is additionally added is also preferable. Disodium edetate, tetrasodium edetate or calcium disodium edetate, and sulfobutylether-β-cyclodextrin, hydroxypropyl-β-cyclodextrin or maltosyl-β-cyclodextrin may be contained in another container.

Usually, the injection of the present invention is substantially free from a non-aqueous solvent (or an aqueous organic solvent) and can be dissolved in or diluted with a solvent whose medium is substantially water. The injection does not need a water-soluble carboxylic acid amide such as nicotinamide and benzamide. Further, the injection of the present invention may be a lyophilized preparation (a lyophilized injection) such as a lyophilized preparation containing about 5 mg to about 5 g of sulfobutylether-β-cyclodextrin, hydroxypropyl-β-cyclodextrin, maltosyl-β-cyclodextrin or a mixture thereof, about 0.009 mg to about 20.1 mg of disodium edetate, tetrasodium edetate, calcium disodium edetate or a mixture thereof, about 8 to about 24 mg of N-methylglucamine, and about 1.5 to about 10 mg of sodium hydroxide per 30 mg of the compound represented by the formula (I) such as lansoprazole, its optically active compound or a salt thereof; and the lyophilized preparation containing further about 10 to about 100 mg (preferably about 50 to about 70 mg) of mannitol. Disodium edetate, tetrasodium edetate or calcium disodium edetate, and sulfobutylether-β-cyclodextrin, hydroxypropyl-β-cyclodextrin or maltosyl-β-cyclodextrin may be contained in a container different from the container in which the other components are contained. Even such lyophilized preparation can be dissolved in at least one liquid or solvent selected from the group consisting of water for injection (distilled water for injection), an infusion solution containing an electrolytic solution (physiological saline etc.), a nutrient infusion and the like, and can easily be formed into an injectable solution. As the container, a glass container and a plastic container can be used.

The present invention is useful as a method for preventing or treating peptic ulcer, gastroesophageal reflux disease; gastritis; Zollinger-Ellison syndrome; NUD (Non Ulcer Dyspepsia); gastric cancer; gastric MALT lymphoma; upper gastrointestinal hemorrhage due to gastric ulcer, duodenal ulcer, acute stress ulcer and acute gastric mucosal lesion, ulcer caused by a nonsteroidal anti-inflammatory agent; gastric hyperacidity and ulcer due to postoperative stress; upper gastrointestinal hemorrhage due to invasive stress; atrophic gastritis after endoscopic mucosal resection (EMR) for early gastric cancer; hyperplastic gastric polyp; idiopathic thrombocytopenic purpura; a disease caused by *Helicobacter pylori;* asthma caused by gastric acid reflux, sleep disorder due to gastric acid reflux; abdominal pain due to GERD; laryngitis; chronic obstructive pulmonary disease (COPD); obstructive apnea; and Barrett's esophagus, which comprises administering the injection to humans.

Further, the present invention also discloses use for prevention or treatment of peptic ulcer, gastroesophageal reflux disease; gastritis; Zollinger-Ellison syndrome; NUD (Non Ulcer Dyspepsia); gastric cancer; gastric MALT lymphoma; upper gastrointestinal hemorrhage due to gastric ulcer, duodenal ulcer, acute stress ulcer and acute gastric mucosal lesion, ulcer caused by a nonsteroidal anti-inflammatory agent; gastric hyperacidity and ulcer due to postoperative stress; upper gastrointestinal hemorrhage due to invasive stress; atrophic gastritis after endoscopic mucosal resection (EMR) for early gastric cancer; hyperplastic gastric polyp; idiopathic thrombocytopenic purpura; a disease caused by *Helicobacter pylori*; asthma caused by gastric acid reflux, sleep disorder due to gastric acid reflux; abdominal pain due to GERD; laryngitis; chronic obstructive pulmonary disease (COPD); obstructive apnea; and Barrett's esophagus.

As used herein, the term "an injection" refers to not only a final injectable solution, but also to an injection precursor which can be prepared into a final injectable solution with the use of a dissolving solution when used (for example, a liquid injection (a concentrated or condensed injection) or a solid injection (such as a lyophilized injection)) and further to the injection precursor contained in a container.

Preferably, the injection of the present invention contains the compound represented by the formula (I) such as lansoprazole, its optically active compound or a salt thereof, and cyclodextrin or a derivative thereof in an amount corresponding to about 0.16- to about 170-fold by weight, preferably about 0.16- to about 100-fold by weight relative to lansoprazole, its active compound or a salt thereof, and a chelating agent in an amount corresponding to about 0.03% to about 67% by weight, preferably about 0.3% to about 33% by weight, more preferably about 0.6% to about 6.7% by weight relative to the compound represented by the formula (I), its active compound or a salt thereof.

The "chelating agent" may be edetic acid, its salt or a derivative thereof, phosphoric acid or its salt, or citric acid or its salt as long as it is capable of forming a complex compound with a metal ion. The salt is preferably a pharmaceutically acceptable salt including, for example, a salt with an inorganic base, such as alkali metal salt (e.g., a sodium salt, a potassium salt), an alkaline earth metal salt (e.g., a calcium salt, a magnesium salt), and an ammonium salt. The salt may also be a salt with an organic base, a salt with a basic amino acid, or the like. In particular, a sodium salt of edetic acid is preferable.

The injection of the present invention can be prepared by using the compound represented by the formula (I) such as lansoprazole, its optically active compound or a salt thereof, and about 0.01 to about 1 equivalent/L, preferably about 0.1 to about 0.6 equivalent/L, more preferably about 0.15 to about 0.25 equivalent/L of a strong alkaline aqueous solution wherein the strong alkali is in an amount of about 0.5 to about 3 equivalents per mol of the formula (I), its optically active compound or a salt thereof and by dissolving the compound represented by the formula (I), its optically active compound or a salt thereof in the strong alkaline aqueous solution. Thus, the present invention also includes the injection obtained by this process for preparation. In this process, the strong alkaline aqueous solution may be a sodium hydroxide aqueous solution.

Thus, the injection of the present invention has an effect of suppressing to form insolubles from fatty acid salts or the like even when the injectable solution prepared by adding cyclodextrin or a derivative thereof is kept or supplied in any container. The solubility of the compound represented by the formula (I) such as lansoprazole, its optically active compound or a salt thereof can be improved by further adding a strong alkali while decreasing the amount of the strong alkali to be used. Thus, according to the present invention, a pain and a local irritation caused by injection is improved by preparing the injection by using the compound represented by the formula (I), its optically active compound or a salt thereof and a strong alkali without using a non-aqueous solvent (or a water-soluble organic solvent) wherein the strong alkali is in an amount of about 0.5 to about 3 equivalents per mol of the compound represented by the formula (I), its optically active compound or a salt thereof. In addition, the solubility of the freeze-dried preparation in at least one liquid selected from water for injection, infusion solutions and nutrient infusions can be improved by preparing a freeze-dried preparation by using the compound represented by the formula (I), its optically active compound or a salt thereof and a strong alkali without using a non-aqueous solvent (or a water-soluble organic solvent) wherein the strong alkali is in an amount of about 0.5 to about 3 equivalents per mol of the formula (I), its optically active compound or a salt thereof.

The injection according to the present invention may further contain N-methylglucamine (meglumine). The amount of "N-methylglucamine" to be incorporated is about 0.1 to about 1 mg, preferably about 0.1 to about 0.8 mg per mg of the compound represented by the formula (I), its optically active compound or a salt thereof.

The lowering of pH can be prevented by addition of N-methylglucamine because of the buffer capacity of N-methylglucamine, thereby preventing deterioration of the quality of a preparation due to precipitation of impurities or the like. Further, by incorporating N-methylglucamine, pH as high as about 9 to about 11 or so can be maintained, and higher pH of about 8 to about 11 or so can be maintained depending on the concentration.

The "injection" of the present invention may further contain a saccharide. The "saccharide" includes, for example, a monosaccharide (e.g., glucose, galactose, ribose, xylose, mannose, maltotriose, maltotetraose), a disaccharide (e.g., sucrose, lactose, cellobiose, trehalose, maltose), a trisaccharide (e.g., raffinose), a sugar alcohol (e.g., sorbitol, inositol, mannitol), a polysaccharide (e.g., dextran, chondroitin sulfate, hyaluronic acid, dextrin sulfate) and a salt thereof (e.g., sodium chondroitin sulfate, sodium hyaluronate). Among the saccharides, a sugar alcohol is preferred. Mannitol is particularly preferred.

The amount of the "saccharide" to be incorporated is about 0.1 to about 20 mg, preferably about 0.5 to about 10 mg (e.g., about 1 to about 10 mg) per mg of the compound represented by the formula (I), its optically active compound or a salt thereof.

The injection of the present invention may further contain additive(s).

The "additive" includes, for example, a pH regulator such as a water-soluble inorganic acid (e.g., hydrochloric acid, sulfuric acid, carbonic acid, phosphoric acid), an alkali metal salt of a water-soluble inorganic acid (e.g., sodium chloride, potassium chloride, sodium sulfate, potassium sulfate), an alkaline earth metal salt of a water-soluble inorganic acid (e.g., calcium chloride, magnesium chloride), a water-soluble organic acid (e.g., citric acid, tartaric acid, lactic acid, succinic acid, malic acid, acetic acid, oxalic acid, benzoic acid, tannic acid, gluconic acid, fumaric acid, sorbic acid, erysorbic acid, mesylic acid, mefenamic acid), an alkali metal salt of a water-soluble organic acid (e.g., sodium citrate, sodium tartrate), an alkaline earth metal salt of a water-soluble organic acid (e.g., calcium citrate, calcium lactate, magnesium gluconate), a neutral amino acid (e.g., glycine, alanine), an acidic amino acid (e.g., aspartic acid, glutamic acid), a salt of an acidic amino acid (e.g., sodium aspartate, potassium glutamate) and a salt of a basic amino acid (e.g., lysine hydrochloride, arginine hydrochloride).

If necessary, the "injection" according to the present invention can comprise a buffer (e.g., sodium dihydrogen phosphate, disodium hydrogen phosphate), a tonicity agent (e.g., glucose, sodium chloride), a stabilizer (e.g., sodium hydrogen sulfite), a soothing agent (e.g., glucose, benzyl alcohol, mepivacaine hydrochloride, xylocaine hydrochloride, procaine hydrochloride, carbocaine hydrochloride) and a preservative (e.g., p-oxybenzoate ester such as methyl p-oxybenzoate and propyl p-oxybenzoate, thimerosal, chlorobutanol, benzyl alcohol).

Specific examples of the injection according to the present invention include an injection comprising the compound represented by the formula (I) such as lansoprazole, its optically active compound or a salt thereof, cyclodextrin or a derivative thereof, a chelating agent, a strong alkali (e.g., an alkali metal hydroxide such as sodium hydroxide) and N-methylglucamine, and the injection further comprising a saccharide. The preferred injection includes an injection comprising the compound represented by the formula (I), its optically active compound or a salt thereof, sodium hydroxide, a β-cyclodextrin derivative, a salt of edetatic acid and N-methylglucamine, and the injection further comprising mannitol. The amount of the respective components in such an injection may be as follows: about 5 mg to 5 g of sulfobutylether-β-cyclodextrin, hydroxypropyl-β-cyclodextrin, maltosyl-β-cyclodextrin or a mixture thereof; about 0.009 mg to about 20.1 mg of disodium edetate, tetrasodium edetate, calcium disodium edetate or a mixture thereof; about 8 mg to about 24 mg of N-methylglucamine and about 1.5 mg to about 10 mg of sodium hydroxide per 30 mg of the compound represented by the formula (I), its optically active compound or a salt thereof. Further, about 10 to about 100 mg (preferably about 50 to about 70 mg) of a sugar alcohol (e.g., mannitol) may also be added. β-cyclodextrin and a salt of edetic acid may be provided in a container different from the container in which the other components are contained and mixed with the other components when used.

The injection of the present invention may be in a liquid form (e.g., in the form of an aqueous injectable solution), or may be in a semi-solid form (e.g., a concentrated aqueous injection) or in a solid form. The preferred injection of the present invention is a lyophilized preparation (a lyophilized injection). The injection according to the present invention also includes an injection to be dissolved in or diluted with a dissolving solution or a diluent when used. The pH of the injection according to the present invention is adjusted to about 8 to 12, preferably about 9 to 12, when used.

The injection of the present invention (in particular, a lyophilized preparation) can be dissolved in or diluted with a dissolving solution or a diluent (for example, an aqueous dissolving solution or diluent such as water for injection such as distilled water for injection and an infusion solution (e.g., an electrolyte liquid such as physiological saline)) substantially free from a non-aqueous solvent (e.g., a water-soluble organic solvent such as propylene glycol and polyethylene glycol) to prepare easily the injectable solution. Thus, usually the injection of the present invention is substantially free from a non-aqueous solvent (e.g., a water-soluble organic solvent such as propylene glycol and polyethylene glycol). Moreover, in such an aqueous injection (e.g., an injectable solution), the solubility of the compound represented by the formula (I) such as lansoprazole, its optically active compound or a salt thereof is not deteriorated even when the solvent is substantially water (e.g., distilled water). Further, the injection of the present invention may be dissolved in a non-aqueous solvent if necessary.

Because an aqueous solution of N-methylglucamine has a sufficient buffer capacity at pH of about 9 to about 11, the lowering of pH of a solution containing the compound represented by the formula (I), its optically active compound or a salt thereof can be suppressed during the production of the injection comprising the compound represented by the formula (I), its optically active compound or a salt thereof and during re-dissolving the injection, thereby preventing the deterioration of its quality.

The injection of the present invention can be prepared by dissolving the compound represented by the formula (I) such as lansoprazole, its optically active compound or a salt thereof in an aqueous strong alkali solution (e.g., an aqueous sodium hydroxide solution), then adding β-cyclodextrin or a derivative thereof and preferably a chelating agent, and filling the solution into a vial or an ampoule, and if necessary, lyophilizing the solution. When N-methylglucamine, a saccharide, an additive and the like are added, the injection can be obtained by dissolving the compound represented by the formula (I), its optically active compound or a salt thereof, β-cyclodextrin or a derivative thereof, the chelating agent, N-methylglucamine, the saccharide, the additive and the like in an aqueous strong alkali solution (e.g., an aqueous sodium hydroxide solution, etc.) and filling the solution into a vial or an ampoule, and if necessary, lyophilizing the solution. The injection may be prepared by filling a chelating agent and β-cyclodextrin or its derivative in a container different from the container in which the other components are contained.

The most preferred concentration of the "aqueous strong alkali solution" is about 0.15 to about 0.25 equivalent/L. In other words, for example, when sodium hydroxide is employed as the strong alkali, the concentration of the "aqueous sodium hydroxide solution" is about 0.15 to about 0.25 mol/L. When a strong alkali other than sodium hydroxide is employed as the strong alkali, the injection of the present invention can be also prepared according to the above method.

The "dissolving" of the compound represented by the formula (I) such as lansoprazole, its optically active compound or a salt thereof in an aqueous strong alkali solution may be carried out by methods which are known per se.

The "lyophilization" may be carried out by methods which are known per se, and is desirably carried out generally by freezing a solution at a temperature of -25°C or lower, and drying the frozen product with elevating the shelf temperature to 25-40°C, while retaining a degree of vacuum in a drying oven at about 13.3 Pa or less.

As the "glass container (vial)", one made of a glass material available for an injection is preferred. The preferred "vial" includes USP TYPE I, II, or III, particularly TYPE I. A glass vial from which an alkali is eluted in a lower amount than usual can also be used.

A plastic vial such as a vial made of cyclic polyolefin (e.g., CZ Vial manufactured by Daikyo Seiko, Ltd.) is also employed.

The shape and size of the vial are not particularly limited. The capacity of the vial is preferably 100 mL or less, more preferably 40 mL or less, and particularly 20 mL or less. Typical examples of the vial include, for example, 17P vial, 9P vial, 5P vial, and 3.5P vial.

When an "ampoule" is used, an ampoule made of a glass material available for an injection is preferred as a glass container, and an ampoule made of polyethylene, polypropylene, a polyethylene-polypropylene copolymer, polyvinyl chloride, an ethylene-vinyl acetate copolymer, an ethylene-propylene copolymer, silicone, polybutadiene, a thermoplastic elastomer, Teflon (Registered Trademark), polyurethane, cyclic polyolefin or polyolefin can be used as a plastic container. The shape and size of the ampoule are not particularly limited. The capacity of the ampoule is preferably 30 mL or less, more preferably 20 mL or less, and particularly 10 mL or less. Typical examples of the ampoule include, for example, 10P ampoule, 5P ampoule, and 3P ampoule.

Further, a pre-filled syringe wherein the injection is previously filled in a syringe may be also provided.

A container for the injection can be coated with a packaging film. The packaging film is not particularly limited, and as an example, the packaging film includes cellophane, cellophane coated with vinylidene chloride, polyethylene, oriented polypropylene coated with vinylidene chloride, nylon, oriented nylon, oriented nylon coated with vinylidene chloride, oriented polypropylene, non-oriented polypropylene, polyester, polyester coated with vinylidene chloride, aluminum, ethylene-vinyl alcohol polymer, and the like. The packaging film may be transparent or colored. Further, the packaging film may have a light blocking effect and may have an effect to block light of a specific wavelength range which promotes photo-degradation. Preferable examples of such a film include a film having an effect to block ultraviolet light and visible light. The light blocking-film material is not particularly limited, includes materials which can block light of an intended wavelength range, and may contain an ultraviolet absorber. A light blocking effect by paper may be provided. The packaging film may also have an oxygen-blocking effect and may contain an oxygen absorber. Further, the packaging film may have heat resisting properties so that it can be pasteurized or sterilized. Furthermore, the film may have fine pores so as to enhance gas permeability, and the gas permeability may be adjusted by the film thickness or the number of pores. The film may be adhered, closely adhered, or bonded to a container by means of heating or adhering.

In the case where the injection of the present invention is a lyophilized preparation and it takes long time for the solution of the injection to become transparent due to vigorous foaming of the contents upon re-dissolution, the re-dissolving time can be reduced by using a vial or ampoule coated with silicone. The silicone used for coating includes silicone oil such as a poly(dimethylsiloxane) and poly(methylhydrogensiloxane); varnish silicone such as methyl varnish silicone and methyl phenyl varnish silicone. As one example of the preferred silicone, there may be mentioned KM-740 (manufactured by Shin-Etsu Chemical Co., Ltd.).

In the case where the injection of the present invention is an aqueous injection, the injection in a vial or an ampoule can be used by pulling out a predetermined amount of the injection via an syringe from the vial or the ampoule. In the case where the injection of the present invention is a lyophilized preparation, the preparation is utilized by re-dissolving when used.

As to the "solvent for re-dissolving", it is unnecessary to employ a solution containing a non-aqueous solvent such as polyethylene glycol that might exhibit toxicity is feared when used in a high concentration. Examples of the solvent for re-dissolving include water for injection (distilled water for injection), an infusion solution [an electrolyte solution (e.g., physiological saline, a Ringer's solution), a nutrition infusion solution (a carbohydrate solution (e.g., a glucose solution such as 5% (w/v) glucose solution, etc.), an injectable solution of a protein amino acid, an injectable solution of a vitamin, etc.), a blood substitute wherein an electrolyte solution and a nutrition infusion solution (e.g., a carbohydrate solution) are combined, a fat emulsion wherein fats are emulsified, etc.], and a mixed solvent of two or more of them. To the solvent may be optionally added a pH-adjusting agent (e.g., an acidic substance, a weak-alkaline substance) or the like. The injection of the present invention may be re-dissolved in an organic solvent such as ethanol, propylene glycol and polyethylene glycol, and after dissolving in the organic solvent, the injection may be further diluted with a solvent as exemplified with respect to the above "solvent for re-dissolving".

The above "electrolyte solution" is a solution obtained by dissolving an electrolyte in water for injection, and includes, for example, a solution comprising one or more of sodium chloride, potassium chloride, calcium chloride, sodium lactate, sodium dihydrogen phosphate, magnesium carbonate and the like, a lactated Ringer's solution, an acetated Ringer's solution, and the like. The preferred electrolyte solution includes a solution containing sodium chloride, particularly physiological saline (0.9% (w/v) sodium chloride solution).

The above "carbohydrate solution" is a solution obtained by dissolving a saccharide in water for injection, and includes, for example, a solution containing one or more of glucose, fructose, sorbitol, mannitol, dextran and the like. The preferred carbohydrate solution includes 5 to 70% (w/v) glucose solution, especially 5% (w/v) glucose solution and 10% (w/v) glucose solution.

The above "injectable solution of a protein amino acid" is a solution obtained by dissolving an amino acid in water for injection, and includes, for example, a solution containing one or more of glycine, aspartic acid, lysine and the like.

The above "injectable solution of a vitamin" is a solution obtained by dissolving a vitamin in water for injection, and includes, for example, a solution containing one or more of vitamin B₁, vitamin C and the like.

Preferred example of the "solvent for re-dissolving" includes water for injection, physiological saline, and a glucose solution (e.g., 5% (w/v) glucose solution).

The compound represented by the formula (I) such as lansoprazole, its optically active compound or a salt thereof has an excellent anti-ulcer action, a gastric acid secretion-inhibiting action, a mucosa-protecting action, an anti-Helicobacter *pylori* action, etc., and is of lower toxicity.

The injection of the present invention is useful in mammals (e.g., humans, non-human animals such as monkeys, sheep, bovines, horses, dogs, cats, rabbits, rats and mice) as an agent for prevention or treatment of peptic ulcer (e.g., gastric ulcer, duodenal ulcer, anastomotic ulcer, acute stress ulcer); gastroesophageal reflux disease [GERD; e.g., reflux esophagitis, gastroesophageal reflux disease without esophagitis (Symptomatic GERD)]; gastritis; Zollinger-Ellison syndrome (which is often included in peptic ulcer); NUD (Non Ulcer Dyspepsia); gastric cancer (including gastric cancer associated with enhanced production of interleukin-1β due to genetic polymorphism of interleukin-1); gastric MALT lymphoma; upper gastrointestinal hemorrhage due to gastric ulcer, duodenal ulcer, acute stress ulcer and acute gastric mucosal lesion, ulcer caused by a nonsteroidal anti-inflammatory agent [including ulcer due to aspirin (lower dose for preventing heart disease)]; gastric hyperacidity and ulcer due to postoperative stress; upper gastrointestinal hemorrhage due to invasive stress (stress caused by major surgery which requires intensive control after the surgery, and by cerebrovascular disorder, head trauma, multiple organ failure and extensive burn which require intensive care); atrophic gastritis after endoscopic mucosal resection (EMR) for early gastric cancer; hyperplastic gastric polyp; idiopathic thrombocytopenic purpura; or a disease caused by *Helicobacter pylori* (NUD, GERD, atrophic gastritis after endoscopic mucosal resection (EMR) for early gastric cancer, hyperplastic gastric polyp, idiopathic thrombocytopenic purpura, iron-deficiency anemia, chronic urticaria, Raynaud's phenomenon, ischemic heart disease, migraine headache, Guillan-Barre' syndrome, etc. due to *Helicobacter* pylori); asthma caused by gastric acid reflux, sleep disorder due to gastric acid reflux; abdominal pain due to GERD; laryngitis; chronic obstructive pulmonary disease (COPD); obstructive apnea; and Barrett's esophagus. Particularly, the injection is useful for the treatment of gastroesophageal reflux disease (GERD); and gastric ulcer, duodenal ulcer, acute stress ulcer and acute gastric mucosal lesion, etc. each of which involves haemorrhagia which is impossible to be treated by oral administration. Further, the injection of the present invention is also useful for *Helicobacter pylori* eradication; suppression of the above-mentioned upper gastrointestinal hemorrhage; treatment and prevention of gastric hyperacidity and ulcer due to postoperative stress; pre-anesthetic administration, etc. Particularly, the injection is useful for the prevention and treatment of gastric ulcer, duodenal ulcer, acute stress ulcer and acute gastric mucosal lesion each of which involves haemorrhagia which is impossible to be treated by oral administration of the compound represented by the formula (I) such as lansoprazole, its optical active compound or a salt thereof. Further, the injection is also useful for the prevention and treatment of gastroesophageal reflux disease (GERD). The injection of the present invention can be administered parenterally (e.g., drip administration, intravenous administration, intramuscular administration, subcutaneous administration) for the purpose of treating or preventing these diseases. When the injection of the present invention is parenterally administered to the subject to whom oral administration cannot be applied because of hemorrhage, the injection of the present invention exhibits superior hemostatic effects by parenteral administration, and once oral administration becomes possible, such parenteral administration can be replaced by oral administration.

The compound represented by the formula (I) such as lansoprazole, its optically active ingredient or a salt thereof which is the active ingredient in the injection of the present invention may be used in combination with other active ingredients (e.g., one to three other active ingredients).

The "other active ingredients" include, for example, substances having an anti-Helicobacter *pylori* activity, imidazole compounds, bismuth salts, quinolone compounds and so forth. Among these substances, preferred are substances having an anti-Helicobacter *pylori* activity, imidazole compounds, etc. The "substances having an anti-*Helicobacter pylori* activity" include, for example, penicillin antibiotics (e.g., amoxicillin, benzylpenicillin, piperacillin, mecillinam), cefem antibiotics (e.g., cefixime, cefaclor), macrolide antibiotics (e.g., erythromycin antibiotics such as erythromycin, clarithromycin), tetracycline antibiotics (e.g., tetracycline, minocycline, streptomycin), aminoglycoside antibiotics (e.g., gentamicin, amikacin), imipenem, and so forth. Among these substances, preferred are penicillin antibiotics and macrolide antibiotics. The "imidazole compounds" include, for example, metronidazole and miconazole. The "bismuth salts" include, for example, bismuth acetate and bismuth citrate. The "quinolone compounds" include, for example, ofloxacin, ciproxacin. In particular, it is preferred for *Helicobacter pylori* eradication that the injection of the present invention is used in combination with penicillin antibiotics (e.g., amoxicillin) and/or erythromycin antibiotics (e.g., clarithromycin).

The dose per day of the compound represented by the formula (I) such as lansoprazole, its optically active ingredient or a salt thereof which is the active ingredient in the injection of the present invention varies depending on severity of symptom; age, sexuality and body weight of a recipient; time and interval of administration; type of active ingredients, etc., and is not particularly limited. For example, the dose per day is about 0.1 to about 2 mg, preferably about 0.2 to about 1.5 mg of the compound represented by the formula (I) or its optically active compound per kg of body weight, when parenterally administered as an anti-peptic ulcer agent to an adult human (60 kg). The injection of the present invention is administered once a day or dividedly twice or thrice per day. The concentration of the compound represented by the formula (I), its optical active compound or a salt thereof in the injection to be administered is about 0.001 to about 40 mg/mL, preferably about 0.01 to about 30 mg/mL, particularly preferably about 0.03 to about 10 mg/mL.

The injection of the present invention has an excellent quality in that the injection is free from the formation of insoluble particulate matters when the pharmaceutical composition containing the compound represented by the formula (I) such as lansoprazole that is a 2-[(2-pyridinyl)methylsulfinyl]benzimidazole compound, its optically active compound or a salt thereof is filled, kept and supplied in a glass or plastic container.

The following examples further specifically illustrate the present invention but are not to be construed as limiting the scope of the invention.

As mannitol used in the following examples, the one that complies with the Japanese Pharmacopoeia, 14th Edition, European Pharmacopoeia and USP was used.

### Example 1

2-[[[3-Methyl-4-(2,2,2-trifluoroethoxy)-2-pyridinyl] methyl] sulfinyl]-1H-benzimidazole (lansoprazole; hereinafter briefly referred to as Compound A) and disodium edetate were rapidly dissolved in 0.2 mol/L aqueous sodium hydroxide solution, and then mannitol, N-methylglucamine, sulfobutylether-β-cyclodextrin and water for injection were added. After dissolving them, the resultant solution was filtered with Durapore Filter (0.22 µm) (manufactured by Nihon Millipore Corporation). The solution thus obtained (2 mL) was filled in a 17P vial (manufactured by Daiwa Special Glass, Co., Ltd.) and lyophilized to prepare a lyophilized preparation for injection containing 30 mg of Compound A, 1.5 mg of disodium edetate, 3.82 mg of sodium hydroxide, 60 mg of mannitol, 10 mg of N-methylglucamine and 300 mg of sulfobutylether-β-cyclodextrin sodium salt (trade name: CAPTISOL, manufactured by CyDex Inc.) (the lyophilized preparation for injection is hereinafter briefly referred to as Preparation A).

The pharmaceutical preparation shown in Table 1 was dissolved in 5 mL of physiological saline to prepare an injectable solution. The pH of and foreign insoluble matter in each injectable solution were measured. The foreign insoluble matter was measured in accordance with the Japanese Pharmacopoeia, General Tests, Foreign Insoluble Matter Test for Injection. The results are shown in Table 2.

A solution obtained by dissolving the pharmaceutical preparation shown in Table 1 in 5 mL of physiological saline showed a pH value of about 11 and met the criteria of foreign insoluble matter for injection provided by the Japanese Pharmacopoeia. Thus, it was found that the lyophilized injection of the present invention is also excellent in qualities as an injection.

**Table 1**

| | |
|---|---|
| Compound A | 30 mg |
| N-methylglucamine | 10 mg |
| Mannitol | 60 mg |
| Sodium hydroxide | 3.82 mg |
| Disodium edetate | 1.5 mg |
| Sulfobutylether-β-cyclodextrin sodium salt | 300 mg |

**Table 2**

| | pH | Foreign insoluble matter (just after dissolution) |
|---|---|---|
| Preparation A | 10.8 | Clear and free from foreign insoluble matters that is clearly detectable |

The pharmaceutical preparation shown in Table 1 was diluted with 50 mL of physiological saline in an infusion container made of polyvinyl chloride (0.9% Sodium Chloride Injection USP in Mini-Bag Plus Container from Baxter Healthcare Corporation) and then left at 25°C. 0, 4, 8, 12, 18 and 24 hours after dilution, the pH of and insoluble particulate matter in each injectable solution were measured. The insoluble particulate matter was measured in accordance with the Japanese Pharmacopoeia, General Tests, Insoluble Particulate Matter Test for Injection, Method 1, Light Obscuration Particle Count Test. The results are shown in Table 3.

**Table 3**

| | Time after dilution | pH | Measured value of insoluble particulate matter (Number of particles per container) | |
|---|---|---|---|---|
| | | | Particles equal to or greater than 10 µm | Particles equal to or greater than 25 µm |
| Preparation A | 0 hour | 10.2 | 787 | 13 |
| | 4 hours | 10.1 | 427 | 0 |
| | 8 hours | 10.1 | 253 | 7 |
| | 12 hours | 10.1 | 83 | 7 |
| | 18 hours | 10.1 | 260 | 3 |
| | 24 hours | 10.1 | 70 | 3 |

In a plastic container made of polyvinyl chloride used in U.S.A., the formation of particulate matter in Preparation A of the present invention was suppressed, and over 24 hours after dilution, the number of insoluble particulate matter was sufficiently lower than the number as regulated for injection in the Japanese pharmacopoeia that the number of insoluble particulate matter which is equal to or greater than 10 µm is not more than 6,000 and the number of insoluble particulate matter which is equal to or greater than 25 µm is not more than 600 per container. Thus, it was found that the injection of the present invention can be provided as an injection of higher qualities when used in a plastic container.

### Industrial Applicability

The injection of the present invention can be provided as a high-quality injection in which foreign insoluble matter or insoluble particulate matter is not formed even when the injection containing a 2-[(2-pyridinyl)methylsulfinyl]benzimidazole compound such as lansoprazole useful as an anti-ulcer agent, its optically active compound or a salt thereof is kept and supplied in a plastic container as well as in a glass container.

## Claims

1. An injection comprising a compound represented by formula (I): 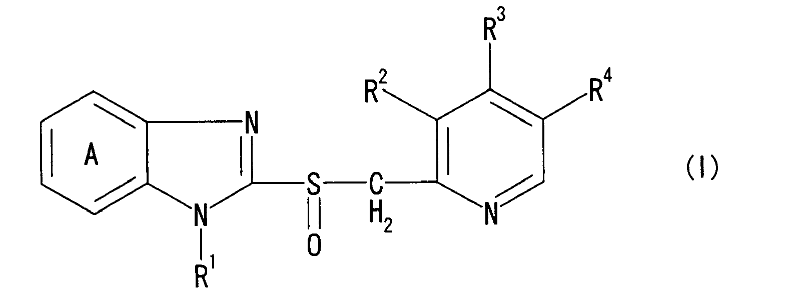 wherein the ring A is a benzene or pyridine ring which may have a substituent, R¹ represents a hydrogen atom, an aralkyl group which may have a substituent, an acyl group or an acyloxy group, and R², R³ and R⁴ are the same or different and each represent a hydrogen atom, an alkyl group which may have a substituent, an alkoxy group which may have a substituent, or an amino group which may have a substituent, or its optically active compound or a salt thereof, in combination with a sulfobutylated derivative of β-cyclodextrin or a salt thereof, wherein pH of the injection is 8 to 12 when used.

2. The injection according to claim 1, wherein the compound of the formula (I) is 2-[[[3-methyl-4-(2,2,2-trifluoroethoxy)-2-pyridinyl]methyl]sulfinyl]-1H-benzimidazole (lansoprazole), its optically active compound or a salt thereof.

3. The injection according to claim 1, further comprising a chelating agent.

4. The injection according to claim 1, wherein pH of the injection is 9 to 12 when used.

5. The injection according to claim 1, which comprises about 0.5 to about 3 equivalents of a strong alkali per mol of the compound of the formula (I).

6. The injection according to claim 1, further comprising N-methylglucamine.

7. The injection according to claim 6, wherein the amount of N-methylglucamine is about 0.1 mg to about 1 mg per mg of the compound of the formula (I).

8. An injection substantially free of insolubles which is filled in a container wherein the injection comprises a solution of a compound represented by formula (I): 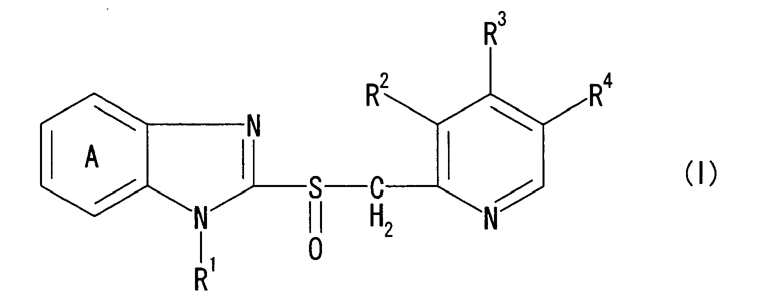 wherein the ring A is a benzene or pyridine ring which may have a substituent, R¹ represents a hydrogen atom, an aralkyl group which may have a substituent, an acyl group or an acyloxy group, and R², R³ and R⁴ are the same or different and each represent a hydrogen atom, an alkyl group which may have a substituent, an alkoxy group which may have a substituent, or an amino group which may have a substituent, or its optically active compound or a salt thereof, and a sulfobutylated derivative of β-cyclodextrin or a salt thereof.

9. The injection according to claim 8, wherein the compound of the formula (I) and the sulfobutylated derivative of β-cyclodextrin or a salt thereof are separately kept, and are mixed with each other when used.

10. The injection according to claim 8, wherein the container is made of polyethylene, polypropylene, a polyethylene-polypropylene copolymer, polyvinyl chloride, an ethylene-vinyl acetate copolymer, an ethylene-propylene copolymer, silicone, polybutadiene, a thermoplastic elastomer, Teflon (Registered Trademark), polyurethane, cyclic polyolefin or polyolefin.

11. The injection according to claim 1, which comprises the sulfobutylated derivative of β-cyclodextrin or a salt thereof in an amount corresponding to about 0.16- to about 170-fold by weight relative to the compound of the formula (I).

12. The injection according to claim 3, wherein the chelating agent consists of edetic acid, its salt or a derivative thereof, phosphoric acid or a salt thereof, or citric acid or a salt thereof.

13. The injection according to claim 3, wherein the chelating agent is a sodium salt of edetic acid.

14. The injection according to claim 3, wherein as the chelating agent, edetic acid or its salt is contained in an amount corresponding to about 0.03% to about 67% by weight relative to the compound of the formula (I).

15. The injection according to claim 1, which is a lyophilized preparation.

16. The injection according to claim 1, which further comprises a saccharide.

17. The injection according to claim 16, wherein the saccharide is a sugar alcohol.

18. The injection according to claim 16, wherein the saccharide is mannitol.

19. The injection according to claim 16, wherein the amount of the saccharide is about 0.1 mg to about 20 mg per mg of the compound of the formula (I).

20. The injection according to claim 2, which comprises about 5 to 5000 mg of the sulfobutylated derivative of β-cyclodextrin or a salt thereof, about 1.5 to about 10 mg of sodium hydroxide, about 8 to about 24 mg of N-methylglucamine, and about 0.009 to about 20.1 mg of disodium edetate per 30 mg of lansoprazole, its optically active compound or a salt thereof.

21. The injection according to claim 2, which comprises about 5 to 5000 mg of the sulfobutylated derivative of β-cyclodextrin or a salt thereof, about 1.5 to about 10 mg of sodium hydroxide, about 8 to about 24 mg of N-methylglucamine, about 10 to about 100 mg of mannitol and about 0.009 to about 20.1 mg of disodium edetate per 30 mg of lansoprazole, its optically active compound or a salt thereof.

22. An injection which is prepared by adding an aqueous or a non-aqueous solvent containing a sulfobutylated derivative of β-cyclodextrin or a salt thereof and edetic acid or its salt to a freeze-dried injection containing 30 mg of lansoprazole, its optically active compound or a salt thereof, about 1.5 to about 10 mg of sodium hydroxide, about 8 to about 24 mg of N-methylglucamine and 60 mg of mannitol.

23. The injection according to claim 1, which is an agent for prevention or treatment of peptic ulcer, gastroesophageal reflux disease; gastritis; Zollinger-Ellison syndrome; NUD (Non Ulcer Dyspepsia); gastric cancer; gastric MALT lymphoma; upper gastrointestinal hemorrhage due to gastric ulcer, duodenal ulcer, acute stress ulcer and acute gastric mucosal lesion, ulcer caused by a nonsteroidal anti-inflammatory agent; gastric hyperacidity and ulcer due to postoperative stress; upper gastrointestinal hemorrhage due to invasive stress; atrophic gastritis after endoscopic mucosal resection (EMR) for early gastric cancer; hyperplastic gastric polyp; idiopathic thrombocytopenic purpura; a disease caused by *Helicobacter pylori;* asthma caused by gastric acid reflux, sleep disorder due to gastric acid reflux; abdominal pain due to GERD; laryngitis; chronic obstructive pulmonary disease (COPD); obstructive apnea; or Barrett's esophagus.

24. A method for preventing or treating peptic ulcer, gastroesophageal reflux disease; gastritis; Zollinger-Ellison syndrome; NUD (Non Ulcer Dyspepsia); gastric cancer; gastric MALT lymphoma; upper gastrointestinal hemorrhage due to gastric ulcer, duodenal ulcer, acute stress ulcer and acute gastric mucosal lesion, ulcer caused by a nonsteroidal anti-inflammatory agent; gastric hyperacidity and ulcer due to postoperative stress; upper gastrointestinal hemorrhage due to invasive stress; atrophic gastritis after endoscopic mucosal resection (EMR) for early gastric cancer; hyperplastic gastric polyp; idiopathic thrombocytopenic purpura; a disease caused by *Helicobacter pylori;* asthma caused by gastric acid reflux, sleep disorder due to gastric acid reflux; abdominal pain due to GERD; laryngitis; chronic obstructive pulmonary disease (COPD); obstructive apnea; or Barrett's esophagus, which comprises administering the injection according to claim 1 to humans.

25. Use of the injection according to claim 1 for an agent for prevention or treatment of peptic ulcer, gastroesophageal reflux disease; gastritis; Zollinger-Ellison syndrome; NUD (Non Ulcer Dyspepsia); gastric cancer; gastric MALT lymphoma; upper gastrointestinal hemorrhage due to gastric ulcer, duodenal ulcer, acute stress ulcer and acute gastric mucosal lesion, ulcer caused by a nonsteroidal anti-inflammatory agent; gastric hyperacidity and ulcer due to postoperative stress; upper gastrointestinal hemorrhage due to invasive stress; atrophic gastritis after endoscopic mucosal resection (EMR) for early gastric cancer; hyperplastic gastric polyp; idiopathic thrombocytopenic purpura; a disease caused by *Helicobacter pylori;* asthma caused by gastric acid reflux, sleep disorder due to gastric acid reflux; abdominal pain due to GERD; laryngitis; chronic obstructive pulmonary disease (COPD); obstructive apnea; or Barrett's esophagus.

26. A method of suppressing the generation of foreign insoluble matter and insoluble particulate matter in an injection filled in a container, which comprises incorporating cyclodextrin or a derivative thereof into the injection, wherein pH of the injection is 7 to 12 when used, and the container is made of polyethylene, polypropylene, a polyethylene-polypropylene copolymer, polyvinyl chloride, an ethylene-vinyl acetate copolymer, an ethylene-propylene copolymer, silicone, polybutadiene, a thermoplastic elastomer, Teflon (Registered Trademark), polyurethane, cyclic polyolefin or polyolefin.
